# FASCICULE DE BREVET EUROPEEN

(11) **EP 1 691 696 B1**
(45) Date de publication et mention de la délivrance du brevet: **12.01.2011**
(21) Numéro de dépôt: 04805779.8
(22) Date de dépôt: 26.10.2004
(51) Int. Cl.: A61B 17/34

(54) **DISPOSITIF DE PRELEVEMENT ET/OU D 'INJECTION DE MOELLE OSSEUSE ET SYSTEME COMPRENANT UN TEL DISPOSITIF**
VORRICHTUNG ZUR ENTFERNUNG UND/ODER INJEKTION VON KNOCHENMARK UND DIESE VORRICHTUNG UMFASSENDES SYSTEM
DEVICE FOR REMOVING AND/OR INJECTING BONE MARROW AND SYSTEM COMPRISING SAID DEVICE

(30) Priorité: 30.10.2003 FR 0312722
(43) Date de publication de la demande: 23.08.2006
(73) Titulaire: Patrice, Richard, 75017 Villa des Ternes (FR)
(72) Inventeur: RICHARD, Patrice, F-75017 Paris (FR); SCHMIT, Christian, F-78124 Mareil sur Mauldre (FR)
(74) Mandataire: CAPRI
(86) Numéro de dépôt international: PCT/FR2004/050536
(87) Numéro de publication internationale: WO 2005/041790

(56) Documents cités:
- WO-A-03/101306
- WO-A-2005/004700
- FR-A- 2 170 919
- GB-A- 1 593 101
- GB-A- 2 130 890
- US-A- 4 256 119
- US-A- 4 641 663
- US-A- 4 922 602
- US-A- 5 322 511
- US-A- 5 330 443
- US-A- 5 478 328
- US-A- 5 597 536
- US-A1- 2003 120 291
- US-A1- 2003 176 811

## Description

La présente invention concerne un dispositif de prélèvement et/ou d'injection de moelle osseuse ainsi qu'un système de prélèvement et/ou d'injection incorporant un tel dispositif.

Un dispositif selon le préambule de la revendication 1 est divulgué dans FR 2 170 919.

Le prélèvement de moelle osseuse est actuellement réalisé à l'aide d'une aiguille creuse (ou trocart) présentant un orifice axial à travers lequel la moelle s'écoule. Ce type de prélèvement nécessite une tige interne (ou mandrin) qui est insérée dans l'aiguille. Cette tige a pour fonction d'éviter toute obturation de l'orifice de l'aiguille, notamment par une esquille d'os lors de l'étape de pénétration de l'aiguille dans l'os. Lors de l'étape de prélèvement en tant que telle, la tige est retirée de l'aiguille et est remplacée par une seringue. L'utilisateur aspire alors la moelle au moyen de cette seringue, via l'orifice de l'aiguille. La moelle aspirée est ensuite transférée dans une poche de collecte et mise en présence d'un anti-coagulant. Ensuite, ces étapes de prélèvement sont réitérées en enfonçant à chaque fois davantage l'aiguille munie de la tige pour recueillir de nouveau de la moelle osseuse. Ce mode de prélèvement présente de nombreux inconvénients. Un inconvénient majeur est lié à la mise en oeuvre d'une succession d'étapes pour aboutir à la quantité souhaitée de moelle pouvant atteindre 1,5 litre. Une séquence typique comprend la mise en place de la tige, la pénétration de l'aiguille dans l'os, le retrait de la tige, la mise en place de la seringue, l'aspiration de la moelle, le retrait de la seringue et la remise en place de la tige. Cette séquence doit être répétée plusieurs fois. La lenteur et la complexité du prélèvement sont des inconvénients relatifs à ce type de prélèvement. En effet, ce type de prélèvement mobilise pendant une à deux heure(s) un nombre important de personnes. Généralement, un ou deux médecin(s) pratique(nt) le prélèvement assisté(s) de un ou deux aide(s) chargé(s) de disposer la moelle osseuse dans des poches de collecte destinées à être stockées. Un autre inconvénient de ce type de prélèvement est relatif aux remplacements successifs de la tige par la seringue aspirant la moelle. Ainsi, le temps de latence s'écoulant entre le retrait de la tige de l'aiguille et la mise en place de la seringue fait que la moelle s'écoulant à travers l'orifice est en contact avec l'air ambiant. Ainsi, ce contact avec l'air ambiant peut être source de contamination microbiologique de la moelle prélevée avec les inconvénients que cella implique par la suite.

La présente invention a donc pour but de fournir un dispositif de prélèvement et/ou d'injection de moelle osseuse qui ne reproduit pas les inconvénients susmentionnés.

La présente invention a également pour but de fournir un tel dispositif pouvant aussi être utilisé dans un mode d'injection par exemple lors d'une transplantation de moelle osseuse d'un donneur à un receveur.

Un autre but de la présente invention est de fournir un dispositif de prélèvement et/ou d'injection qui soit simple d'utilisation, rapide et sure. Plus particulièrement, la présente invention a pour but un dispositif de prélèvement et/ou d'injection requérant moins de personnel, réduisant la durée d'anesthésie des patients et assurant l'obtention d'une moelle de qualité, riche en cellules souches sanguines.

Un autre but de l'invention est d'éviter toute contamination microbiologique de la moelle osseuse en intégrant le dispositif de prélèvement et/ou d'injection selon l'invention dans un système de prélèvement et/ou d'injection stérile.

La présente invention a aussi pour but de fournir de tels dispositif et système qui soient simples et peu coûteux à fabriquer, assembler et utiliser.

La présente invention a donc pour objet un dispositif de prélèvement et/ou d'injection de moelle osseuse selon la revendication 1 et comprenant une zone de préhension, une aiguille présentant au moins un orifice latéral, un manchon protecteur enveloppant au moins partiellement ladite aiguille étant monté de façon mobile par rapport à ladite aiguille entre une position d'obturation dudit au moins un orifice latéral et une position d'ouverture dudit au moins un orifice latéral.

Avantageusement, ledit manchon protecteur du dispositif est monté rotatif autour de l'aiguille.

Avantageusement, ledit manchon protecteur du dispositif comporte au moins une ouverture latérale, se positionnant sensiblement en vis-à-vis dudit au moins un orifice latéral de l'aiguille en position d'ouverture.

Ladite aiguille du dispositif est fixée sur un porte-aiguille, ledit porte-aiguille comprenant des moyens de réception aptes à coopérer avec des moyens de fixation dudit manchon protecteur.

Avantageusement, ledit manchon protecteur du dispositif comporte une première partie constituant un fourreau enveloppant ladite aiguille et une seconde partie comportant lesdits moyens de fixation.

Avantageusement, lesdits moyens de fixation du dispositif comportent au moins une griffe montée pivotante et présentant une surface d'actionnement manuel et une projection.

Avantageusement, lesdits moyens de réception dudit porte-aiguille comportent au moins une rainure apte à recevoir au moins une projection desdits moyens de fixation dudit manchon protecteur.

Avantageusement, ledit dispositif comporte une chambre de mélange reliée à ladite aiguille ainsi qu'à au moins un canal d'entrée et au moins un canal de sortie.

La présente invention a également pour objet un système de prélèvement de moelle osseuse comprenant un tel dispositif.

Avantageusement, ledit système comporte une chambre de mélange reliée à ladite aiguille du dispositif ainsi qu'à au moins un canal d'entrée et au moins un canal de sortie.

Avantageusement, un canal d'entrée est relié à une source de produit anticoagulant.

Avantageusement, un canal de sortie est relié à un réservoir collecteur de moelle.

Avantageusement, ledit système comporte des moyens d'aspiration reliés au moins à ladite aiguille.

Avantageusement, lesdits moyens d'aspiration comportent une pompe à vide.

Avantageusement, lesdits moyens d'aspiration sont commandés par un moyen de commande, tel qu'une pédale actionnée par l'utilisateur.

Avantageusement, ledit canal d'entrée se projette à l'intérieur de la chambre de mélange en direction dudit canal de sortie pour créer un effet venturi.

Avantageusement, ledit système comporte un dispositif temporisateur pour régler la durée des phases d'aspiration de moelle osseuse.

La présente invention a également pour objet un système d'injection de moelle osseuse comprenant un tel dispositif de prélèvement et/ou d'injection.

Avantageusement, ledit dispositif est relié à un réservoir de moelle osseuse, ledit réservoir étant connecté à des moyens de distribution.

Avantageusement, lesdits moyens de distribution comprennent une pompe, telle qu'un pousse-seringue électrique ou une pompe à CO₂.

Avantageusement, ledit système de prélèvement et/ou d'injection est emballé de manière stérile.

D'autres caractéristiques et avantages de l'invention apparaîtront au cours de la description détaillée suivante d'un mode de réalisation particulier de l'invention, faite en référence aux dessins joints donnés à titre d'exemples non limitatifs, et sur lesquels :
- la figure 1 représente une vue schématique en section transversale d'un dispositif de prélèvement et/ou d'injection selon un mode de réalisation avantageux de l'invention,
- la figure 2 représente une vue éclatée en perspective, à échelle agrandie, d'une partie du dispositif de prélèvement et/ou d'injection de la figure 1, et
- la figure 3 représente une vue schématique d'un système de prélèvement et/ou d'injection selon un mode de réalisation de l'invention.

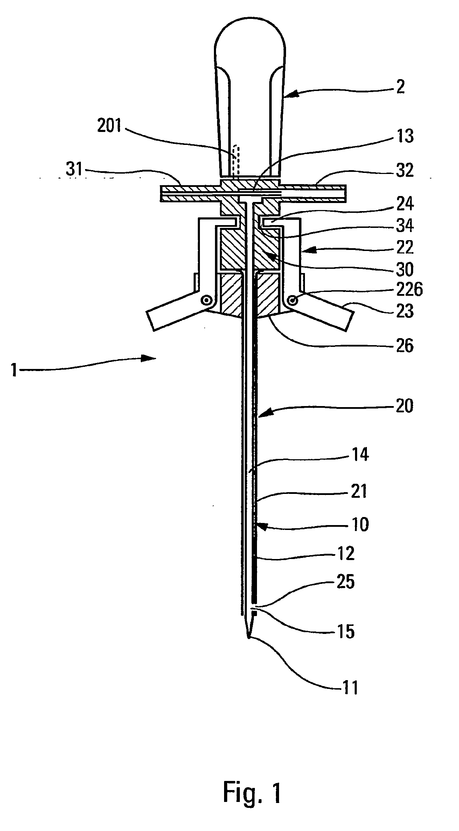

En référence à la figure 1, le dispositif 1 selon l'invention comporte au moins une zone de préhension 2, une aiguille 10 et un manchon protecteur 20. Le dispositif tel que représenté peut servir à la fois au prélèvement et à l'injection de moelle osseuse.

La zone de préhension 2 est une surface destinée à la saisie par l'utilisateur du dispositif lors d'un prélèvement et/ou d'une injection de moelle osseuse. Cette zone de préhension peut par exemple être réalisé sous la forme d'un manche, par exemple un manche similaire à celui d'un de tournevis. Cependant, la forme donnée à ce manche et ces caractéristiques peuvent être quelconques, la présente invention ne se limitant en aucun cas à cette forme de réalisation particulière donnée à titre d'exemple.

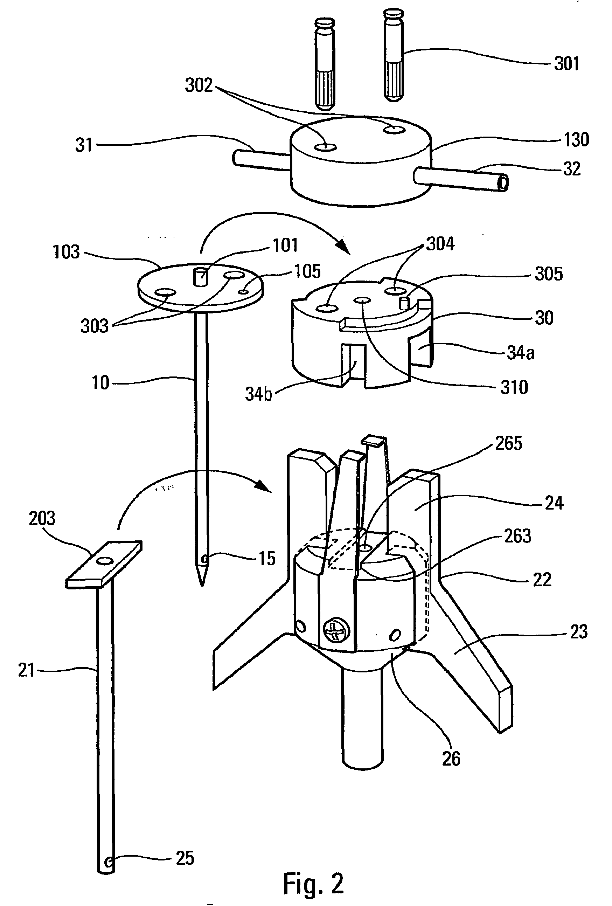

L'aiguille 10 se présente sous la forme d'un corps creux 12 cylindrique se terminant par une pointe 11 adaptée à percer l'os. Cette aiguille présente un conduit d'écoulement 14. Selon l'invention, l'aiguille comporte au moins un orifice latéral 15, avantageusement deux, ménagé près de la pointe 11, avantageusement à l'extrémité du corps cylindrique 12. Il peut être noté que les orifices ménagés sur le corps de l'aiguille peuvent se présenter sous une forme quelconque. L'aiguille 10 coopère avantageusement avec un porte-aiguille 30. Celui-ci peut être monobloc avec l'aiguille, ou, comme représenté sur la figure 2, être réalisé séparément en étant démontable et coopérer par emboîtement comme cela sera plus amplement décrit ci-après.

Le manchon protecteur 20 comprend avantageusement d'une part une première partie formant un fourreau 21 et d'autre par une seconde partie comportant des moyens de fixation 22. Le fourreau 21 constitue une gaine avantageusement cylindrique enveloppant totalement ou partiellement l'aiguille 10. Ainsi, l'aiguille peut être enveloppée sur seulement une partie de sa longueur ou sur seulement une partie de sa largeur, à condition que ledit fourreau coopère avec ledit au moins un orifice latéral 15 de l'aiguille. Le manchon 20 est, selon l'invention, monté de manière mobile par rapport à l'aiguille 10 entre une position d'obturation dudit au moins un orifice latéral 15 et une position d'ouverture dudit au moins un orifice latéral 15. Avantageusement, le fourreau 21 comprend au moins une ouverture latérale 25 apte à être placée en correspondance avec un orifice latéral 15 respectif de l'aiguille, en position d'ouverture. Cette ouverture latérale 25 présente de préférence une forme correspondant à celle donnée à l'orifice latéral 15 de l'aiguille, mais une forme différente est aussi envisageable. Ainsi, diverses combinaisons de formes peuvent être envisagées permettant de mettre en vis-à-vis le ou les orifice(s) latéral(aux) 15 de l'aiguille et la ou les ouverture(s) latérale(s) 25 du fourreau pour ainsi définir un ou plusieurs passage(s) que la moelle osseuse empruntera lors de son prélèvement ou de son injection.

Les moyens de fixation 22 du manchon protecteur 20 présentent avantageusement une forme globale d'ailettes ou de griffes. Selon un mode de réalisation préféré de l'invention, le dispositif comprend deux moyens de fixation 22 situés sensiblement en vis-à-vis. Les moyens de fixation 22 peuvent comporter d'une part une surface d'actionnement manuel 23 et d'autre part une projection 24. Les moyens de fixation 22 sont avantageusement montés sur une douille de fixation 26. Cette douille de fixation 26 et le fourreau 21 du manchon protecteur 20 pourraient être réalisés de manière monobloc. Toutefois dans l'exemple de la figure 2, ces deux éléments sont réalisés séparément en étant démontables et coopèrent par emboîtement comme cela sera plus amplement décrit ci-après. Chaque moyen de fixation 22 peut former un levier apte à pivoter autour d'un axe respectif 226 entre une position de fixation et une position de libération. La surface d'actionnement manuel 23 desdits moyens de fixation se projette avantageusement vers l'extérieur afin de faciliter la manipulation du manchon protecteur 20 par l'utilisateur. Les projections 24 servent de surface d'accrochage dudit manchon protecteur 20 au dispositif selon l'invention. Dans le mode de réalisation représenté, les projections 24 des moyens de fixation 22 coopèrent avec des moyens de réception 34. Ces moyens de réception 34 se présentent avantageusement sous la forme de rainures ménagées radialement au niveau du porte-aiguille 30. Le porte-aiguille 30 peut former avec la zone de préhension (ou manche) 2 un ensemble monobloc. Les rainures 34 peuvent être au nombre de quatre répartis en deux couples 34a, 34b de deux rainures chacun. Chaque couple 34a, 34b comporte deux rainures opposées l'une à l'autre afin de permettre à ces rainures de coopérer avec deux griffes 22, également opposées l'une à l'autre. Ainsi, le manchon protecteur étant monté mobile par rapport à l'aiguille 10, les projections 24 seront positionnées au niveau de l'un ou l'autre couple de rainure 34 selon la position choisie (obturation ou ouverture). Le déplacement du manchon protecteur 20 se fait de préférence par rotation du manchon protecteur 20 autour de l'aiguille 10. Pour ce faire, l'utilisateur dégage les projections 24 des griffes 22 du premier couple de rainures 34a, fait tourner le manchon protecteur 20 autour de l'aiguille 10, puis positionne les projections 24 des griffes 22 dans l'autre couple de rainures 34b. Ainsi, dans la première position dite d'ouverture chaque orifice latéral 15 de l'aiguille est disposé face à une ouverture latérale respective 25 du manchon protecteur. Ce positionnement aboutit alors à la création d'un passage pour le prélèvement et/ou l'injection de moelle osseuse. Dans la seconde position dite d'obturation aucun orifice latéral 15 de l'aiguille n'est ouvert et est au contraire obturé par le fourreau 21 du manchon 20. Ainsi, dans ce cas, aucun passage n'est défini pour prélever et/ou injecter de la moelle osseuse interdisant donc tous prélèvements ou injections. Cette deuxième position correspond donc à celle utilisée pour faire pénétrer l'aiguille 10 dans l'os. De cette manière, il n'y a aucun risque de boucher le ou les orifices 15 de l'aiguille 10 lors de l'insertion de celle-ci dans l'os.

Il est à noter que le mode de réalisation décrit ci-dessus constitue une forme de réalisation avantageuse de l'invention, et différentes variantes sont envisageables. Par exemple, le déplacement du manchon protecteur 20 de la position d'obturation à la position d'ouverture (et vice-versa) pourrait se faire par un déplacement axial du manchon protecteur ou par une combinaison mettant en jeu une rotation et un déplacement axial. De même, les moyens de fixation 22 du manchon 20 peuvent être réalisés différemment, et incorporer par exemple des moyens de repérage pour indiquer de manière claire à l'utilisateur la position du manchon par rapport à l'aiguille.

Selon un mode de réalisation préféré de l'invention, le dispositif comprend une chambre de mélange 13. Cette chambre de mélange 13 est reliée au conduit 14 de l'aiguille, à au moins un canal d'entrée 31 et à au moins un canal de sortie 32. Le conduit 14 de l'aiguille apporte la moelle osseuse prélevée. Ledit au moins un canal d'entrée 31 peut apporter un anti-coagulant, tel que de l'héparine, servant à éviter la formation de caillots sanguins au niveau de la moelle osseuse prélevée. Ledit au moins un canal de sortie 32 contient alors un mélange d'anti-coagulant et de moelle osseuse, et peut mener vers un réservoir collecteur. Ces canaux d'entrée 31 et de sortie 32 sont disposés avantageusement de part et d'autre de la chambre de mélange 13. En variante, il peut être envisagé que la chambre de mélange et lesdits au moins un canal d'entrée et au moins un canal de sortie soient réalisé séparément du dispositif de prélèvement et/ou d'injection 1.

Ledit au moins un canal d'entrée 31 se projette avantageusement à l'intérieur de la chambre de mélange 13 et peut même se positionner directement au niveau dudit au moins canal de sortie 32. Ce type de disposition favorise la création d'un effet venturi ayant pour conséquence une meilleure homogénéisation du mélange anti-coagulant et moelle osseuse au niveau dudit au moins un canal de sortie 32. Cette meilleure homogénéisation participe donc à l'obtention d'une moelle de meilleure qualité.

La figure 2 présente un mode de réalisation de l'invention dans lequel la chambre de mélange 13 est formée dans un élément rapporté 130 coopérant avec le porte aiguille 30. Dans ce mode de réalisation, des chevilles de fixation 301 sont insérées dans des trous 302, 303, 304 respectivement ménagés sur ledit élément rapporté 130, l'aiguille 10 et le porte-aiguille 30 afin d'obtenir un ensemble monobloc. Il peut être également prévu que ces chevilles 301 coopèrent avec des trous 201 prévus dans le manche 2 pour le fixer à cet ensemble. Selon ce mode de réalisation, l'aiguille 10 peut être pourvue d'une plateforme 103 permettant le maintien de l'aiguille sur le porte aiguille 30. Cette plateforme est positionnée de telle manière à laisser une partie libre 101 de l'aiguille 10 rejoindre la chambre de mélange 13. Cette partie libre met donc en contact le conduit 14 de l'aiguille avec la chambre de mélange 13.

Afin de faciliter la mise en place de l'aiguille 10 sur le porte-aiguille 30, un moyen d'indexation peut être prévu. Ce moyen d'indexation peut par exemple être composé d'un trou 105 ménagé au niveau de la plate forme 103 de l'aiguille coopérant avec une protubérance 305 du porte-aiguille. Ainsi, lors de l'introduction de l'aiguille 10 au niveau d'un orifice central 310 du porte-aiguille, la plateforme sera alors positionnée d'une façon telle que la protubérance 305 s'engage dans le trou 105. Il résulte de ce positionnement un alignement des trous 303 et 304 facilitant ainsi l'insertion des chevilles de fixation 301. De même, un moyen d'indexation pour la mise en place de l'élément rapporté 130 au niveau de la plateforme 103 peut être prévu afin d'aligner les trous 302 avec les trous 303 et 304.

Comme décrit précédemment, le fourreau 21 du manchon protecteur peut être une pièce rapportée. Dans ce cas, le fourreau 21 est introduit au niveau d'un orifice 265 traversant la douille de fixation 26. Le manchon protecteur 20 est retenu grâce à une bride 203 se positionnant dans un logement de forme complémentaire 263 ménagé au niveau de la douille de fixation.

Le mode de réalisation décrit ci-dessus en référence aux figures 1 et 2 est avantageux en ce que le dispositif est facile à assembler et à démonter. Le dispositif peut donc être emballé de manière démontée et stérile dans un emballage commun. Lors de l'utilisation, l'utilisateur insère le fourreau 21 du manchon 20 dans l'orifice 265 de la douille 26. Il place ensuite l'aiguille 10 dans le porte-aiguille 30, et cet ensemble est assemblé sur la douille 26, l'aiguille 10 passant dans le fourreau 21.

Eventuellement, la chambre de mélange 13 prévue dans l'élément rapporté 130 peut être assemblée sur le porte aiguille 30 au moyens des cheville 301, avant assemblage du porte-aiguille 30 sur la douille 26. De même, le manche 2 peut être pré-assemblé avec lesdites chevilles 301.

Bien entendu, cette mise en oeuvre est un exemple. En particulier, divers éléments décrits ci-dessus pourraient être réalisés de manière monobloc pour limiter le nombre de pièces à assembler. Par exemple, la chambre de mélange 13 pourrait être réalisée dans le porte-aiguille 30, et l'aiguille 10 pourrait être monobloc avec ledit porte-aiguille 30. La chambre de mélange 13 pourrait aussi être réalisée dans le manche 2. Le manchon 20 pourrait aussi être réalisé de manière monobloc. L'essentiel est de réaliser un manchon 20 déplaçable par rapport à l'aiguille 10 entre ses positions d'obturation et d'ouverture.

La présente invention concerne également un système de prélèvement et/ou un système d'injection intégrant un dispositif de prélèvement et/ou d'injection 1 tel que précédemment décrit.

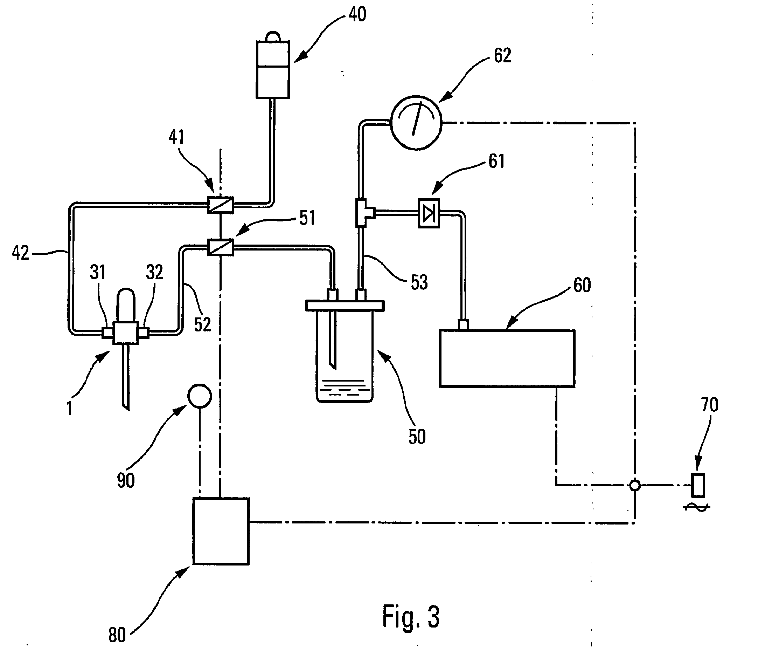

La figure 3 présente une forme de réalisation préférée du système de prélèvement. Selon cette forme de réalisation, un canal d'entrée 31 du dispositif est relié à une source d'anti-coagulant 40, par l'intermédiaire d'une tubulure 42. Un canal de sortie 32 est lui connecté à un réservoir collecteur 50 de moelle osseuse par l'intermédiaire d'une tubulure 52. Des électrovannes 41 et 51 peuvent être respectivement placées au niveau des tubulures 42 et 52 afin de contrôler respectivement l'apport d'anti-coagulant et la quantité prélevée de moelle osseuse mélangée à l'anti-coagulant. Le réservoir collecteur 50 est de préférence en relation avec des moyens d'aspiration 60. Ces moyens d'aspiration peuvent être formée par une pompe à vide reliée à une connexion électrique 70. Cette pompe à vide créée une dépression entraînant l'anti-coagulant et la moelle osseuse prélevée au niveau du réservoir collecteur 50. En variante, on peut utiliser une pompe péristaltique ou tout autre moyen d'aspiration approprié. Le système est commandé avantageusement par un moyen de commande unique contrôlant l'ouverture et la fermeture des électrovannes 41 et 51. Ce moyen de commande 90 peut être une pédale actionnée par l'utilisateur. Un dispositif anti-retour 61 peut être placé au niveau d'une tubulure 53 reliant le réservoir collecteur 50 aux moyens d'aspiration 60. Ce dispositif anti-retour 61 a pour but d'empêcher toute aspiration de mélange d'anti-coagulant et de moelle osseuse du collecteur 50 vers les moyens d'aspiration 60. Un vacuostat 62 peut être également intégré au système de prélèvement. Ce vacuostat 62 peut alors intervenir dans la commande de l'ouverture des électrovannes 41 et 51, à un seuil de vide poussé prédéterminé préférentiellement inférieur à 900 millibars. Ainsi, ce vacuostat 62 permet d'obtenir une aspiration efficace et forte dès l'ouverture des vannes favorisant un meilleur décollement des progéniteurs hématopoïétiques et donc l'obtention d'une moelle plus riche. En outre, ce système peut intégrer un temporisateur 80 ayant pour fonction de régler, notamment limiter, la durée du prélèvement de la moelle osseuse. D'autres moyens de réglage pour déterminer certaines caractéristiques du prélèvement sont envisageables. Parmi ces caractéristiques, on peut noter outre la durée, l'intensité, le débit, la fréquence, etc.

La présente invention concerne également un système d'injection utilisable dans la transplantation de moelle osseuse au niveau des travées osseuses d'os spongieux. Dans ce cas, le dispositif 1 est simplement relié à un réservoir de moelle osseuse de type seringue. Cette seringue peut être actionnée manuellement ou être reliée à des moyens de distribution type pompe, par exemple unpousse-seringue électrique ou tout autre sorte de pompe, telle qu'une pompe à CO₂, ou à tout autre système de commande qu'il soit électrique ou non. Ces moyens assurent ainsi une maîtrise totale de la vitesse et de la quantité de moelle osseuse à transplanter.

Lorsque le dispositif est utilisé dans un système d'injection, le canal d'entrée 31 de la chambre de mélange 13 est de préférence fermé. Par exemple, ce canal 31 peut être retiré et le trou résultant bouché par tout moyen adapté. La chambre de mélange 13 devient alors avantageusement une simple chambre de transit pour la moelle.

Le dispositif de prélèvement et/ou d'injection ainsi que le système de prélèvement et/ou d'injection selon l'invention sont avantageusement fournis en kit. Les pièces qui composent ce kit sont avantageusement emballées de manière stérile évitant ainsi tout contamination microbiologique.

Bien que l'invention ait été décrite en référence à un mode de réalisation particulier de celle-ci, il est entendu qu'elle n'est pas limitée par celui-ci, mais qu'au contraire, l'utilisateur peut y apporter toutes modifications utiles sans sortir du cadre de la présente invention tel que défini par les revendications annexées.

## Revendications

1. Dispositif de prélèvement et/ou d'injection de moelle osseuse (1) comprenant:
- une zone de préhension (2),
- une aiguille (10) présentant au moins un orifice latéral (15) et étant fixée sur un porte-aiguille (30), et en ce qu'un manchon protecteur (20) enveloppe au moins partiellement ladite aiguille (10), ledit manchon protecteur (20) étant monté de façon mobile par rapport à ladite aiguille (10) entre une position d'obturation dudit au moins un orifice latéral (15) et une position d'ouverture dudit au moins un orifice latéral (15), **caractérisé en ce que** ledit porte-aiguille (30) comprenant des moyens de réception (34) aptes à coopérer avec des moyens de fixation (22) dudit manchon protecteur (20) afin de fixer ledit manchon protecteur dans la position d'obturation et dans la position d'ouverture.

2. Dispositif (1) selon la revendication 1, dans lequel ledit manchon protecteur (20) est monté rotatif autour de l'aiguille (10).

3. Dispositif (1) selon la revendication 1 ou 2, dans lequel ledit manchon protecteur (20) comporte au moins une ouverture latérale (25), se positionnant sensiblement en vis-à-vis dudit au moins un orifice latéral (15) de l'aiguille (10) en position d'ouverture.

4. Dispositif (1) selon la revendication 1, dans lequel ledit manchon protecteur (20) comporte une première partie constituant un fourreau (21) enveloppant ladite aiguille et une seconde partie comportant lesdits moyens de fixation (22).

5. Dispositif (1) selon la revendication 1 ou 4, dans lequel lesdits moyens de fixation (22) comportent au moins une griffe montée pivotante et présentant une surface d'actionnement manuel (23) et une projection (24).

6. Dispositif (1) selon l'une quelconque des revendications 1, 4 ou 5, dans lequel lesdits moyens de réception (34) dudit porte-aiguille (30) comportent au moins une rainure apte à recevoir au moins une projection (24) desdits moyens de fixation (22) dudit manchon protecteur (20).

7. Dispositif (1) selon l'une quelconque des revendications précédentes, dans lequel ledit dispositif comporte une chambre de mélange (13) reliée à ladite aiguille ainsi qu'à au moins un canal d'entrée (31) et au moins un canal de sortie (32).

8. Dispositif (1) selon la revendication 7, dans lequel ladite chambre de mélange est prévue dans le porte-aiguille (30).

9. Dispositif (1) selon la revendication 7, dans lequel ladite zone de préhension (2) comporte un manche, ladite chambre de mélange étant prévue dans ledit manche.

10. Dispositif selon la revendication 7, comprenant un élément rapporté (130) entre ladite zone de préhension (2) et ledit porte-aiguille (30), ladite chambre de mélange étant prévue dans ledit élément rapporté (130).

11. Système de prélèvement de moelle osseuse **caractérisé en ce qu'**il comprend un dispositif (1) selon l'une quelconque des revendications précédentes.

12. Système de prélèvement selon la revendication 11, dans lequel ledit système comporte une chambre de mélange (13) reliée à ladite aiguille (10) du dispositif ainsi qu'à au moins un canal d'entrée (31) et au moins un canal de sortie (32).

13. Système de prélèvement selon la revendication 11 ou 12, dans lequel un canal d'entrée (31) est relié à une source de produit anticoagulant (40).

14. Système de prélèvement selon l'une quelconque des revendications 11 à 13, dans lequel un canal de sortie (32) est relié à un réservoir collecteur de moelle (50).

15. Système de prélèvement selon l'une quelconque des revendications 11 à 14, dans lequel ledit système comporte des moyens d'aspiration reliés au moins à ladite aiguille (10).

16. Système de prélèvement selon la revendication 15 dans lequel lesdits moyens d'aspiration (60) comportent une pompe à vide.

17. Système de prélèvement selon la revendication 15 ou 16 dans lequel lesdits moyens d'aspiration (60) sont commandés par un moyen de commande (90), tel qu'une pédale actionnée par l'utilisateur.

18. Système de prélèvement selon l'une quelconque des revendications 11 à 17, dans lequel ledit canal d'entrée (31) se projette à l'intérieur de la chambre de mélange (13) en direction dudit canal de sortie (32) pour créer un effet venturi.

19. Système de prélèvement selon l'une quelconque des revendications 11 à 18, dans lequel ledit système comporte un dispositif temporisateur (80) pour régler la durée des phases d'aspiration de moelle osseuse.

20. Système d'injection de moelle osseuse, **caractérisé en ce qu'**il comprend un dispositif (1) selon l'un quelconque des revendications 1 à 10.

21. Système d'injection selon la revendication 20, dans lequel ledit dispositif (1) est relié à un réservoir de moelle osseuse, ledit réservoir étant connecté à des moyens de distribution.

22. Système d'injection selon la revendication 20 ou 21, dans lequel lesdits moyens de distribution comprennent une pompe, telle qu'un pousse-seringue électrique ou une pompe à CO₂.

23. Système de prélèvement et/ou d'injection selon l'une quelconque des revendications 11 à 22, dans lequel ledit système est emballé de manière stérile.

## Claims

1. A bone-marrow extraction and/or injection device (1) comprising:
· a grip zone (2); and
· a needle (10) presenting at least one side orifice (15) and being fastened onto a needle holder (30), and in that a protective sleeve (20) surrounds at least part of said needle (10), said protective sleeve being mounted to move relative to said needle (10) between a closed position of said at least one side orifice (15) and an open position of said at least one side orifice (15), the device being **characterized in that** said needle holder (30) includes reception means (34) that are suitable for co-operating with fastener means (22) of said protective sleeve (20) so as to fix said protective sleeve in the closed position and in the open position.

2. A device (1) according to claim 1, in which said protective sleeve (20) is mounted to turn about the needle (10).

3. A device (1) according to claim 1 or claim 2, in which said protective sleeve (20) includes at least one side opening (25) that is positioned substantially facing said at least one side orifice (15) of the needle (10) in the open position.

4. A device (1) according to claim 1, in which said protective sleeve (20) comprises a first portion constituting a sheath (21) surrounding said needle, and a second portion comprising said fastener means (22).

5. A device (1) according to claim 1 or claim 4, in which said fastener means (22) include at least one claw that is pivotally mounted, and that presents a manual actuation surface (23) and a projection (24).

6. A device (1) according to any one of claims 1, 4 or 5, in which said reception means (34) of said needle holder (30) comprise at least one groove that is suitable for receiving at least one projection (24) of said fastener means (22) of said protective sleeve (20).

7. A device (1) according to any preceding claim, in which said device includes a mixing chamber (13) that is connected to said needle, and to at least one inlet channel (31) and at least one outlet channel (32).

8. A device (1) according to claim 7, in which said mixing chamber is provided in the needle holder (30).

9. A device (1) according to claim 7, in which said grip zone (2) comprises a handle, said mixing chamber being provided in said handle.

10. A device according to claim 7, including an add-on element (130) between said grip zone (2) and said needle holder (30), said mixing chamber being provided in said add-on element (130).

11. A bone-marrow extraction system, **characterized in that** it includes a device (1) according to any preceding claim.

12. An extraction system according to claim 11, in which said system includes a mixing chamber (13) that is connected to said needle (10) of the device, and to at least one inlet channel (31) and at least one outlet channel (32).

13. An extraction system according to claim 11 or claim 12, in which an inlet channel (31) is connected to a source (40) of anticoagulant.

14. An extraction system according to any one of claims 11 to 13, in which an outlet channel (32) is connected to a bone-marrow collection vessel (50).

15. An extraction system according to any one of claims 11 to 14, in which said system includes suction means connected at least to said needle (10).

16. An extraction system according to claim 15, in which said suction means (60) comprise a vacuum pump.

17. An extraction system according to claim 15 or claim 16, in which said suction means (60) are controlled by control means (90), such as a pedal that is actuated by the user.

18. An extraction system according to any one of claims 11 to 17, in which said inlet channel (31) projects into the mixing chamber (13) and towards said outlet channel (32), so as to create a Venturi effect.

19. An extraction system according to any one of claims 11 to 18, in which said system includes a timer device (80) for setting the duration of the bone-marrow suction stages.

20. A bone-marrow injection system, **characterized in that** it includes a device (1) according to any one of claims 1 to 10.

21. An injection system according to claim 20, in which said device (1) is connected to a bone-marrow reservoir, said reservoir being connected to dispenser means.

22. An injection system according to claim 20 or claim 21, in which said dispenser means comprise a pump, such as a syringe with an electrically-driven plunger, or a CO₂ pump.

23. An extraction and/or injection system according to any one of claims 11 to 22, in which said system is packaged in sterile manner.

## Patentansprüche

1. Vorrichtung zur Entnahme und/oder Injektion von Knochenmark (1), aufweisend:
- eine Greifzone (2),
- eine Nadel (10), die mindestens eine seitliche Öffnung (15) aufweist und auf einem Nadelhalter (30) befestigt ist, und eine Schutzhülse (20), die die Nadel (10) zumindest teilweise umhüllt, wobei die Schutzhülse (20) in Bezug auf die Nadel (10) zwischen einer Schließposition der mindestens einen seitlichen Öffnung und einer Öffnungsposition der mindestens einen seitlichen Öffnung (15) verschiebbar gelagert ist, **dadurch gekennzeichnet, dass** der Nadelhalter (30) Aufnahmemittel (34) aufweist, die mit den Befestigungsmitteln (22) der Schutzhülse (20) zusammenwirken können, um die Schutzhülse in der Schließposition und in der Öffnungsposition zu fixieren.

2. Vorrichtung (1) nach Anspruch 1, wobei die Schutzhülse (20) drehbar um die Nadel (10) gelagert ist.

3. Vorrichtung (1) nach Anspruch 1 oder 2, wobei die Schutzhülse (20) mindestens eine seitliche Öffnung (25) aufweist, die sich in der Öffnungsposition in etwa gegenüber der mindestens einen seitlichen Öffnung (15) der Nadel (10) positioniert.

4. Vorrichtung (1) nach Anspruch 1, wobei die Schutzhülse (20) einen ersten Teil, der eine die Nadel umhüllende Hülle (21) darstellt, und einen zweiten Teil, der die Befestigungsmittel (22) enthält, aufweist.

5. Vorrichtung (1) nach Anspruch 1 oder 4, wobei die Befestigungsmittel (22) mindestens einen Greifer aufweisen, der schwenkbar gelagert ist und eine manuelle Betätigungsfläche (23) sowie einen Überstand (24) aufweist.

6. Vorrichtung (1) nach einem der vorhergehenden Ansprüche 1, 4 oder 5, wobei die Aufnahmemittel (34) des Nadelhalters (30) mindestens eine Nut aufweisen, die mindestens einen Überstand (24) der Befestigungsmittel (22) der Schutzhülse (20) aufnehmen kann.

7. Vorrichtung (1) nach einem der vorhergehenden Ansprüche, wobei die Vorrichtung eine Mischkammer (13) aufweist, die derart mit der Nadel verbunden ist, dass mindestens ein Einlasskanal (31) und mindestens ein Auslasskanal (32) gebildet sind.

8. Vorrichtung (1) nach Anspruch 7, wobei die Mischkammer in dem Nadelhalter (30) vorgesehen ist.

9. Vorrichtung (1) nach Anspruch 7, wobei die Greifzone (2) einen Griff aufweist, wobei die Mischkammer in dem Griff vorgesehen ist.

10. Vorrichtung nach Anspruch 7, aufweisend ein Anbauelement (130) zwischen der Greifzone (2) und dem Nadelhalter (30), wobei die Mischkammer in dem Anbauelement (130) vorgesehen ist.

11. System zur Entnahme von Knochenmark, **dadurch gekennzeichnet, dass** es eine Vorrichtung (1) nach einem der vorhergehenden Ansprüche aufweist.

12. Entnahmesystem nach Anspruch 11, wobei das System eine Mischkammer (13) aufweist, die derart mit der Nadel (10) der Vorrichtung verbunden ist, dass mindestens ein Einlasskanal (31) und mindestens ein Auslasskanal (32) gebildet sind.

13. Entnahmesystem nach Anspruch 11 oder 12, wobei ein Einlasskanal (31) mit einer Quelle von gerinnungshemmendem Produkt (40) verbunden ist.

14. Entnahmesystem nach einem der Ansprüche 11 bis 13, wobei ein Auslasskanal (32) mit einem Sammelbehälter für Knochenmark (50) verbunden ist.

15. Entnahmesystem nach einem der Ansprüche 11 bis 14, wobei das System Saugmittel, die zumindest mit der Nadel (10) verbunden sind, aufweist.

16. Entnahmesystem nach Anspruch 15, wobei die Saugmittel (60) eine Vakuumpumpe aufweisen.

17. Entnahmesystem nach Anspruch 15 oder 16, wobei die Saugmittel (60) von einem Steuermittel (90) gesteuert werden, wie einem vom Benutzer betätigten Pedal.

18. Entnahmesystem nach einem der Ansprüche 11 bis 17, wobei sich der Einlasskanal (31) im Inneren der Mischkammer (13) in Richtung zum Auslasskanal (32) erstreckt, um eine Venturiwirkung zu schaffen.

19. Entnahmesystem nach einem der Ansprüche 11 bis 18, wobei das System eine Verzögerungsvorrichtung (80) aufweist, um die Dauer der Knochenmark-Saugphasen zu regulieren.

20. Injektionssystem für Knochenmark, **dadurch gekennzeichnet, dass** es eine Vorrichtung (1) nach einem der Ansprüche 1 bis 10 aufweist.

21. Injektionssystem nach Anspruch 20, wobei die Vorrichtung (1) mit einem Knochenmarkbehälter verbunden ist, wobei der Behälter mit Ausgabemitteln verbunden ist.

22. Injektionssystem nach Anspruch 20 oder 21, wobei die Ausgabemittel eine Pumpe, wie einen elektrischen Spritzenschieber oder eine CO₂-Pumpe, aufweisen.

23. Entnahmesystem und/oder Injektionssystem nach einem der Ansprüche 11 bis 22, wobei das System steril verpackt ist.
